(19)

Europäisches Patentamt
European Patent Office
Office européen des brevets

(11) **EP 4 198 993 A1**

(12) **EUROPEAN PATENT APPLICATION**
published in accordance with Art. 153(4) EPC

(43) Date of publication:
**21.06.2023 Bulletin 2023/25**

(21) Application number: **21855875.7**

(22) Date of filing: **28.07.2021**

(51) International Patent Classification (IPC):
***G16H 10/00*** (2018.01)

(52) Cooperative Patent Classification (CPC):
**G16H 10/00**

(86) International application number:
**PCT/JP2021/027857**

(87) International publication number:
**WO 2022/034796 (17.02.2022 Gazette 2022/07)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.08.2020 JP 2020136492**

(71) Applicant: **CYBERDYNE INC.
Tsukuba-shi, Ibaraki 305-0818 (JP)**

(72) Inventor: **SANKAI, Yoshiyuki
Tsukuba-shi, Ibaraki 305-0818 (JP)**

(74) Representative: **Mewburn Ellis LLP
Aurora Building
Counterslip
Bristol BS1 6BX (GB)**

(54) **LIVING BODY INFORMATION MANAGEMENT SYSTEM AND LIVING BODY INFORMATION MANAGEMENT METHOD**

(57) As a control unit reads active state data, which is stored in a data storage unit, for each specified period of time and transmits it to a server apparatus via a communication network, a subject operates their own mobile terminal at home and activates a device for sensing a desired active state; and after pairing is established, the subject visually checks the active state while always operating functions on a real-time basis.

FIG. 1

1 Biological Information Management System

**Description**

TECHNICAL FIELD

The present invention relates to a biological information management system and a biological information management method and is suited for application, for example, when performing health management by measurements of various vital sensors in everyday life.

BACKGROUND ART

Conventionally, various vital sensors have been being used to manage health conditions in everyday life. In recent years, vital data such as heart beats, pulses, blood pressure, electrocardiogram, and blood oxygen which are measured from a person(s) at home are transmitted to a server on a network and are registered in a database for the server.
With the spread of smartphones in recent years, there is a growing tendency that users desire to integrally manage the various vital sensors by using their own smartphones, and applications regarding the health management are also increasing.
There is proposed a health data management apparatus capable of easily using index data which indicates personal health conditions measured by measurement equipment (see PTL 1). This health data management apparatus is designed, without being connected to a server on a network, so that a user can easily visually recognize the index data, whose icons are displayed on an index panel, while performing database management of the index data.
Furthermore, there is proposed a medical measurement system that has a medical measurement apparatus which measures biological information, and a management device which is carried together with this medical measurement apparatus. The medical measurement system is designed so that at a specified timing which is arbitrarily set after the activation of the medical measurement apparatus, a user ID is authenticated and, if consistency of a plurality of previously recorded user IDs is negative, reading of recorded measurement results is prohibited (see PTL 2).

CITATION LIST

PATENT LITERATURE

PTL 1: Japanese Patent Application Laid-Open (Kokai) Publication No. 2017-12604
PTL 2: Japanese Patent Application Laid-Open (Kokai) Publication No. 2018-59938

SUMMARY OF THE INVENTION

PROBLEMS TO BE SOLVED BY THE INVENTION

Meanwhile, the health data management apparatus of PTL 1 described above only serves a role to receive and update, at a smartphone, the index data based on the measurement results by the measurement equipment by using a smartphone with the purpose of not reading data from the server via the network. Therefore, the user can only easily use the index data, which indicates personal health conditions measured by the measurement equipment, by using the smartphone.
Furthermore, the medical measurement system of PTL 2 described above has the advantage of being capable of suppressing usability degradation and enhancing protection performance of the personal information. However, with this medical measurement system, the medical measurement apparatus which is of a handheld-type only identifies the relevant user by associating the measurement results of a biosensor with the user ID and records them; and it is not designed to use the medical measurement apparatus to perform operations such as activation of the biosensor.
The present invention was devised in consideration of the above-described circumstances and proposes a biological information management system and biological information management method which enable a user to always visually check their own active state, which is personal information, at home on a real-time basis by using their mobile terminal.

MEANS TO SOLVE THE PROBLEMS

In order to solve the above-described problems, there is provided according to the present invention a biological information management system including: an active state sensing unit that senses an active state of at least one or more systems of a subject from among active states of the subject's cognitive system, motor system, nervous system, and physiological system; a mobile terminal capable of communicating with the active state sensing unit via short-range wireless communication; a communication interface unit provided at each of the active state sensing unit and the mobile terminal and designed to establish pairing between the active state sensing unit and the mobile terminal by means of personal authentication; and a server apparatus capable of communicating with the mobile terminal via a communication network, wherein the mobile terminal includes: a control unit that generates a command signal for causing the active state sensing unit to execute a desired function in response to the subject's operation and transmits the command signal to the active state sensing unit with which the pairing is established via the communication interface unit; and a data storage unit that stores active state data which is a sensing result of the active state sensing unit which is received via the communication interface unit; and wherein the control unit reads the active state data, which is stored in the data storage unit, for each specified period and transmits the active state data to the server apparatus via the communication network.

As a result, with the biological information management system, after the subject operates their own mobile terminal at home and activates a device for sensing a desired active state, and then pairing is established, the subject can visually check the active state while always operating functions on a real-time basis.

Moreover, the present invention is designed so that with the mobile terminal, the control unit stores an application according to the active state sensing unit, which is a pairing target, reads the corresponding application data from the active state sensing unit at the time of establishment of the pairing with the active state sensing unit, and activates the application based on the application data.

As a result, with the biological information management system, the subject can perform operations such as the activation of the device which senses the active state of the cognitive system, the motor system, the nervous system, or the physiological system which is selected by the subject as the active state sensing unit, by using their own mobile terminal and can execute the application according to that device by making it reflected in the mobile terminal.

Furthermore, the present invention is designed so that the mobile terminal further includes a display unit that displays a function screen corresponding to the application when with the mobile terminal the control unit activates the application according to the active state sensing unit with which the pairing has been established.

As a result, with the biological information management system, it becomes possible to cause the function screen indicating the application according to the device, which senses the active state desired by the subject, to be displayed on the display unit of their own mobile terminal.

Furthermore, the present invention is designed so that with the mobile terminal, the control unit displays the active state based on the active state data, which is the sensing result of the active state sensing unit received via the communication interface unit, on a real-time basis by combining the active state with the function screen of the display unit.

As a result, with the biological information management system, it becomes possible to display and visually check their own active state on a real-time basis by combining it with the function screen indicating the application according to the device which senses the active state desired by the subject.

Furthermore, the present invention is designed so that with the mobile terminal, the control unit: causes the display unit to display an application for inputting measurement content regarding a body of the subject; and causes the data storage unit to store a measurement result regarding the body of the subject as physical data together with personal information of the subject.

As a result, with the biological information management system, it becomes possible to make effective use of the sensing results of the active state sensing unit at the server apparatus by storing, in the data storage unit, the measurement results regarding the subject's body such as body height, abdominal girth length, and body weight as the physical data by associating them with the subject's personal information.

Furthermore, there is provided according to the present invention a biological information management method when an active state sensing unit that senses an active state of at least one or more systems of a subject from among active states of the subject's cognitive system, motor system, nervous system, and physiological system is made capable of communicating with a mobile terminal via short-range wireless communication and the mobile terminal is made capable of communicating with a server apparatus via a communication network, the biological information management method including: a first step of establishing pairing between the active state sensing unit and the mobile terminal by means of personal authentication via a communication interface unit provided at each of the active state sensing unit and the mobile terminal; a second step executed at the mobile terminal generating a command signal for causing the active state sensing unit to execute a desired function in response to the subject's operation and transmitting the command signal to the active state sensing unit with which the pairing is established via the communication interface unit; a third step executed at the mobile terminal storing active state data, which is a sensing result of the active state sensing unit which is received via the communication interface unit, in a data storage unit; and a fourth step executed at the mobile terminal reading the active state data, which is stored in the data storage unit, for each specified period and transmitting the active state data to the server apparatus via the communication network.

As a result, by the biological information management method, after the subject operates their own mobile terminal at home and activates the device for sensing a desired active state, and then pairing is established, the subject can visually check the active state while always operating functions on a real-time basis.

ADVANTAGEOUS EFFECTS OF THE INVENTION

According to the present invention as described above, it is possible to implement the biological information management system and the biological information management method which enable the user to always visually check their own active state, which is personal information, on a real-time basis at home.

BRIEF DESCRIPTION OF DRAWINGS

Fig. 1 is a block diagram illustrating an overall configuration of a biological information management system according to an embodiment of the present invention;
Fig. 2 is a conceptual diagram illustrating a configuration example of an active state sensing unit;
Fig. 3 shows graphs illustrating pulse waveforms before and after filter application;
Fig. 4 shows conceptual diagrams indicating an ROI of a near-infrared image and an ROI of a far-infrared image;
Fig. 5 shows conceptual diagrams for explaining nasal breathing and mouth breathing;
Fig. 6 shows conceptual diagrams for explaining a temperature status of nasal breathing and mouth breathing;
Fig. 7 shows graphs indicating a change status of a nostril temperature by the nasal breathing and a change status of an oral temperature by the mouth breathing;
Fig. 8 shows graphs on which a breathing method change from the nasal breathing to the mouth breathing and the content of each status are superimpose; and
Fig. 9 shows conceptual diagrams for explaining non-contact measurement of oxygen saturation by using light.

DESCRIPTION OF EMBODIMENTS

An embodiment of the present invention will be described below in detail with reference to the drawings.

(1) Configuration of Biological Information Management System According to The Present Invention

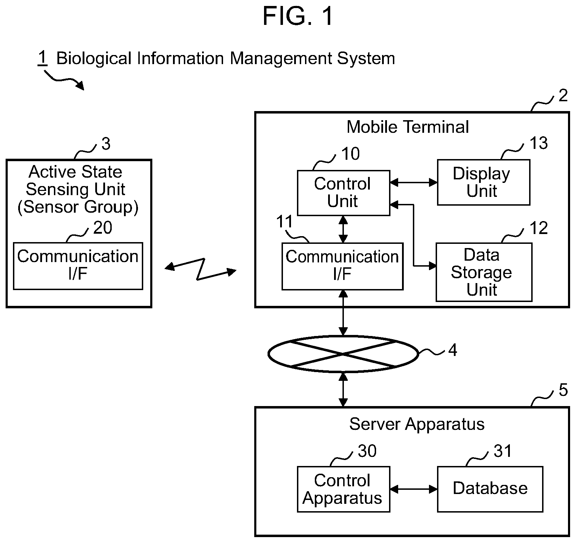

Fig. 1 illustrates a biological information management system 1 according to this embodiment and is configured of a mobile terminal 2 provided for each subject, an active state sensing unit 3 composed of a sensor group capable of sensing the respective active states of the subject's cognitive system, motor system, nervous system, and physiological system, and a server apparatus 5 capable of communicating with the mobile terminal 2 via a communication network 4 such as the Internet.

The mobile terminal 2 is composed of, for example, a smartphone and includes a control unit 10 composed of a CPU (Central Processing Unit) which controls the entire mobile device, a communication interface unit 11 for communicating with the active state sensing unit 3 via short-range wireless communication such as Bluetooth (trade name), and a data storage unit 12 that stores active state data which is the sensing result of the active state sensing unit 3 received via the communication interface unit 11.

The active state sensing unit 3 includes a communication interface unit 20 for communicating with the mobile terminal 2 via the short-range wireless communication. The active state sensing unit 3 is, for example, medical equipment such as a blood pressure meter, a heartbeat sensor, a weight scale, a thermometer, a sleep apnea treatment device (CPAP), a blood sugar level measurement device, and a pacemaker. Furthermore, examples of an active state sensing method include a near-infrared and far-infrared detection method for mainly sensing the active states of the physiological system and the nervous system, a movement recognition method for mainly sensing the active state of the motor system, and a recognition degree sensing method for mainly sensing the active state of the cognitive system as described later.

The biological information management system 1 is designed so that pairing can be established between the active state sensing unit 3 and the mobile terminal 2 via communication interface units 11, 20 which are provided in the active state sensing unit 3 and the mobile terminal 2, respectively.

The data storage unit 12 stores application data corresponding to the active state sensing unit 3, which is composed of a sensor group capable of sensing the respective active states of the subject's cognitive system, motor system, nervous system, and physiological system, with respect to each of the sensors.

With the mobile terminal 2, the control unit 10 generates a command signal for executing a desired function of the active state sensing unit 3 according to the subject's operation and transmits the generated command signal to the active state sensing unit 3 with which the pairing is established via the communication interface unit 11.

When the pairing with the desired active state sensing unit 3 is established, the control unit 10 for the mobile terminal 2 reads an application according to the active state sensing unit 3, which becomes the relevant pairing target, from the data storage unit 12 and activates the application based on the application data.

When the control unit 10 activates the application according to the active state sensing unit 3 with which the pairing is established, it causes the display unit 13 to display a function screen corresponding to the relevant application. This display unit 13 adopts a touch panel system and is designed to display information on a display and also transmit the operation content in response to external input via the display screen (touch operation with a finger or the like) to the control unit 10.

Then, the control unit 10 causes the active state based on the active state data, which is the sensing result of the active state sensing unit 3 received via the communication interface unit 11, to be displayed on a real-time basis by combining it with the function screen of the display unit 13 and causes the active state data to be stored in the data storage unit 12.

The control unit 10 reads the active state data, which is stored in the data storage unit 12, for each specified time period and transmits it to the server apparatus 5 via the communication network 4.

The server apparatus 5 includes: a control apparatus 30 which performs integrated control of the entire apparatus; and a database 31 which stores the active state data, which is received from the mobile terminal 2 via the communication network 4, in a readable manner.

Accordingly, with the biological information management system 1, when a desired active state (the cognitive system, the motor system, the nervous system, or the physiological system) is to be sensed by using the active state sensing unit 3, the subject performs a necessary operation by using their own mobile terminal 2, thereby causing the pairing to be established between the active state sensing unit 3 and the mobile terminal 2 and also causing a desired function according to the above-described operation to be executed.

Then, while visually checking the application corresponding to the active state sensing unit 3 displayed on the display unit 13, the mobile terminal 2 remotely operates the active state sensing unit 3 to cause the desired function to be exhibited and stores the active state data, which is the sensing result of the relevant active state sensing unit 3, in the data storage unit 12 and transmits it to the server apparatus 5 outside via the communication network 4.

Consequently, with the biological information management system 1, after the subject operates their own mobile terminal 2 at home and activates a device for sensing a desired active state and then the pairing is established, the subject can visually check the above-mentioned active state while always operating the function on a real-time basis.

Furthermore, with the mobile terminal 2, the control unit 10 causes the display unit 13 to display an application for inputting measurement content regarding the subject's body and causes the measurement results regarding the subject's body to be stored as physical data, together with the subject's personal information, in the data storage unit 12.

As a result, with the biological information management system 1, it becomes possible for the server apparatus 5 to make effective use of the sensing result of the active state sensing unit 3 by storing the measurement results regarding the subject's body, such as body height, abdominal girth length, and body weight, as physical data in the data storage unit 12 by associating the physical data with the subject's personal information.

(2) Configuration of Active State Sensing Unit (Sensor Group)

The sensor group which constitutes the active state sensing unit 3 is configured from various kinds of sensors capable of sensing the active states of the subject's cognitive system, motor system, nervous system, and physiological system, respectively.

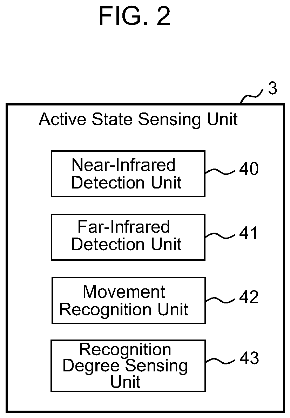

Specifically speaking, as illustrated in Fig. 2, the active state sensing unit 3 includes a sensor group including: a near-infrared detection unit 40 and a far-infrared detection unit 41 which mainly sense the active states of the physiological system and the nervous system; a movement recognition unit 42 which mainly senses the active state of the motor system; and a recognition degree detection unit 43 which mainly senses the active state of the cognitive system.

All of the near-infrared detection unit 40, the far-infrared detection unit 41, the movement recognition unit 42, and the recognition degree sensing unit 43 may be integrated together and be provided in equipment or they may be designed as individual sensors (including cameras) to separately transmit and receive information to and from the mobile terminal 2 via the communication interface unit 20.

(2-1) Method for Sensing Active State of Physiological System

The active state sensing unit 3 includes the near-infrared detection unit 40 and the far-infrared detection unit 41 and measures fluctuations of the subject's pulse rate and changes in their skin temperature in a daily life at the same time and non-invasively by irradiating mainly the subject's face with infrared rays.

The near-infrared detection unit 40 irradiates mainly a region of interest, including cheek parts of the subject's face, with near infrared light and receives the reflected light from the face, thereby generating a near-infrared image.

Moreover, the far-infrared detection unit 41 irradiates mainly the region of interest in the subject's face with far infrared light and detects the skin temperature of the face.

The control unit 10 for the mobile terminal 2 estimates the subject's pulse rate on the basis of a change cycle of intensity waveform in the region of interest among the reflected light received by the near-infrared detection unit 40. The control unit 10 extracts sites which are susceptible to activities of the autonomic nervous system and sites which are hardly susceptible to the activities of the autonomic nervous system, respectively, in the subject's face from the near-infrared image generated by the near-infrared detection unit 40 and calculates a temperature difference in the skin temperature between the respective sites from the detection results of the far-infrared detection unit 41.

(2-1-1) Pulse Measurement Method Based on Infrared Light

Substances with absorbency in biological tissues are water and hemoglobin in blood. The water has the absorption property which is strong against infrared radiation with a wavelength longer than 1350 [nm], while the hemoglobin has the absorption property which is strong against visible light with a wavelength shorter than 650 [nm]. With a noninvasive biological diagnosis using light, light with high biological permeability and of infrared to near-infrared regions with the wavelength of 650 [nm] to 1350 [nm] is often used for irradiation to measure the reflected light and the transmitted light including biological information.

The pulse is a physiological phenomenon where the volume of blood vessels is changed by the work of a blood pressure whose origin is a cardiac output. The volume changes of the blood vessels are caused by changes in a blood flow. The changes in the blood flow cause changes in a light absorption amount by the hemoglobin into the blood, so that the pulse rate can be estimated from a change cycle of the transmitted light and the reflected light of the light used to irradiate the living body.

Since the measurement is performed day and night according to the present invention without requiring the subject to wear measurement equipment, the pulse rate is estimated by using a near infrared camera to measure a change cycle of a reflection ratio of the near infrared light used to irradiate the living body. As the near infrared light is invisible, the measurement can be performed without imposing any burden on a measurement subject even during night. In order to estimate a stressed state from the pulse rate, it is necessary to measure the pulse rate which fluctuates finely as caused by the activities of the autonomic nervous system.

The control unit 10 for the above-mentioned mobile terminal 2 is equipped with an algorithm for extracting a waveform cycle which is necessary to calculate the pulse rate from the waveform data obtained from the near-infrared detection unit 40.

The near-infrared detection unit 40 measures the intensity of the reflected light which is the emitted near infrared rays reflected on the biological tissues. This near-infrared detection unit 40 performs the measurement of a target when resting quietly in bed; and movements of a face surface as caused by minute body movements and breathing are superimposed, as motion artifacts indicating changes in a light quantity of the reflected light, on the pulse waveform.

In order to remove these motion artifacts, firstly a digital filter is applied to a frequency band of the pulse rate. The range of the pulse rate when resting quietly in bed is set as 35 to 180 [bpm] and the motion artifacts may be possibly superimposed also on a bandwidth of 0.5 to 3.0 [Hz] with respect to the measured waveform; however, the waveform which is superimposed on the pulse waveform has a significantly high amplitude.

Therefore, if a distributed value of the waveform data within a certain period of time exceeds a certain threshold value, the control unit 10 recognizes that the subject is performing an action(s), and thereby removes the data during that period of time because it is unreliable. The waveform after band-pass filtering has bimodality attributable to an arterial blood flow. A moving average filter is applied to make it easier to extract feature points so that two crests are reduced to one crest.

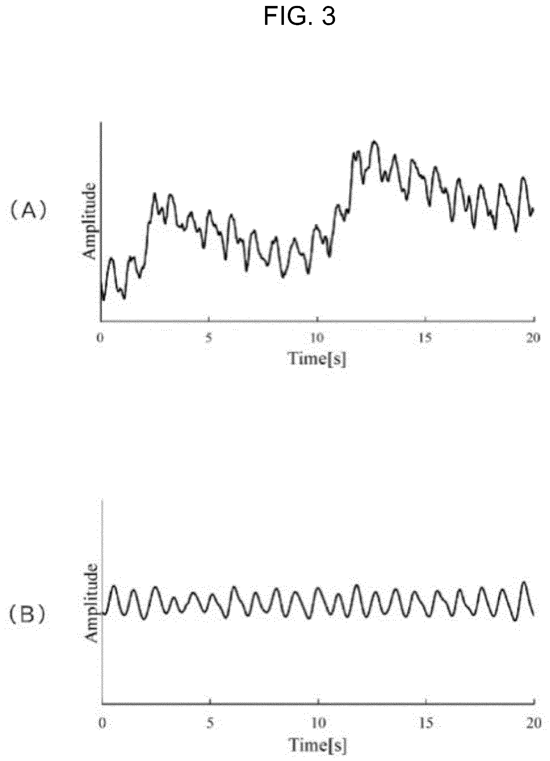

The waveforms before and after applying the band-pass filter and the moving average filter to the measured waveform are indicated, respectively, in Fig. 3(A) and Fig. 3(B). You can see that the motion artifacts superimposed on the pulse waveform have been removed by filtering.

Subsequently, there are generally two major methods which are known as methods for calculating the pulse rate from the light intensity waveform measured with the near infrared camera.

The first method is to set cheeks parts of a face as the region of interest (ROI: Region of Interest) and compare a frequency which is considered as a pulse wave component in a frequency spectrum obtained by a spectrum analyzer with respect to intensity data of the reflected light from the cheek parts for 30 seconds as obtained by the measurement with the RGB camera, with a frequency found from a pulse rate indicated by a pulse oximeter. However, noise caused by body movements is significantly high relative to the pulse waveform. When an attempt is made to calculate the pulse rate by the above-described frequency analysis, it is necessary to greatly restrict activities of the measurement subject.

On the other hand, the second method is to calculate time required for one pulse from a cycle of feature points in a waveform detected from chronological data of the measured waveform and calculate the pulse rate by dividing the above-calculated time by 60. As compared with the method of calculating the pulse rate from the frequency analysis which requires continuous measurement data for a certain period of time, the method of calculating the pulse rate from the measured feature point cycle can remove the motion artifacts by removing the feature point cycle which is falsely detected due to the noise caused by the body movements. Moreover, the required minimum measurement time is shorter than that of the frequency analysis and the pulse rate can be calculated from several pulses, so that it is possible to calculate the pulse rate in shorter time interval.

However, the pulse rate of a healthy person in a state of resting quietly in bed is 60 to 80 [bpm] and the feature point cycles which can be obtained by 5-second measurement are 5 to 7 times, so the problem is that the volume of data which can be obtained is too small to calculate the pulse rate more accurately. Moreover, as can be seen in Fig. 3(A), the waveform of the intensity changes is synchronized with an arterial blood flow and has two crests, which causes false detection of the feature point cycle and becomes a factor to cause an error in the calculated pulse rate.

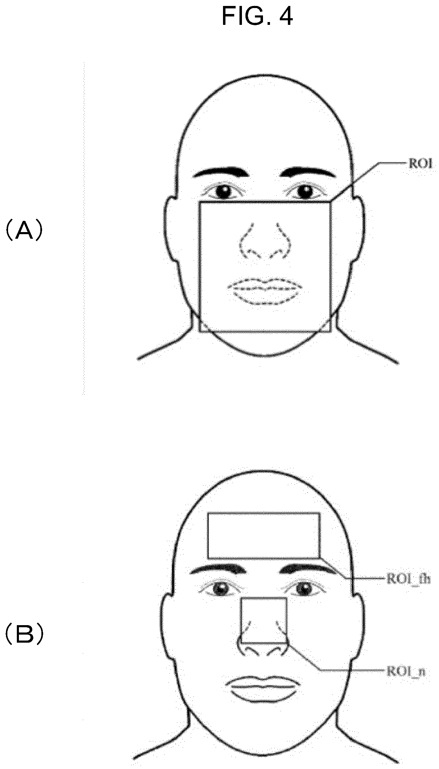

In order to perform daily measurement, a measured site of a living body needs to be exposed day and night. Therefore, the active state sensing unit 3 according to the present invention sets a person's cheek parts as the ROI. Fig. 4(A) illustrates the location of the set ROI. An average of an intensity value measured with pixels inside the set ROI is defined as a measured value and time changes in the measured value are recorded as pulse waveform data. Regarding the feature point cycle of the waveform data, not only top intervals $t_{tp}$ of the waveform data, but also bottom intervals $t_{bp}$ of the wave form data are added as other feature points, thereby increasing the feature points which become reference data for calculating the pulse rate.

Next, the following (a) to (f) indicate algorithms for removing falsely detected $t_{tp}$ and $t_{bp}$ which do not show the pulse cycle, from the detected $t_{tp}$ and $t_{bp}$ in order to extract a necessary feature point cycle to calculate the pulse rate. Under this circumstance, $S_0$ represents a set before the removal.

(a) If a certain peak interval $t_i$ has 30[%] or more fluctuations relative to its immediately preceding peak interval $t_{i-1}$ with respect to each of $t_{tp}$ and $t_{bp}$, that peak interval $t_i$ is removed from the set $S_0$. A set $S_1$ of selected peak intervals can be expressed by Expression (1) indicated below.
[Math. 1]

$$S_1 = \{t_i \mid |t_i - t_{i-1}| < 0.3 t_{i-1}, t_i \epsilon S_0\} \qquad \cdots\cdots (1)$$

(b) A standard deviation $\sigma_0$ of the set $S_0$ is calculated and values of the peak interval $t_i$ which vary within the range of $2\sigma_0$ are removed from the set $S_1$. A set $S_2$ of the selected peak intervals can be expressed by Expression (2) indicated below.
[Math. 2]

$$S_2 = \{t_i \mid |t_i - \tilde{t}| < 2\sigma_0, t_i \epsilon S_1\} \qquad \cdots\cdots (2)$$

(c) A standard deviation $\sigma_2$ of the set $S_2$ is calculated and values of the peak interval $t_i$ which vary within the range of $\sigma_2$ are removed from the set $S_2$. A set $S_3$ of the selected peak intervals can be expressed by Expression (3) indicated below.
[Math. 3]

$$S_3 = \{t_i \mid |t_i - \tilde{t}| < \sigma_2, t_i \epsilon S_2\} \qquad \cdots\cdots (3)$$

(d) A standard deviation $\sigma_3$ of the set $S_3$ is calculated and values of the peak interval $t_i$ which vary within the range of $\sigma_3$ are removed from the set $S_3$. A set $S_4$ of the selected peak intervals can be expressed by Expression (4) indicated below.
[Math. 4]

$$S_4 = \{t_i \mid |t_i - \tilde{t}| < \sigma_3, t_i \epsilon S_3\} \qquad \cdots\cdots (4)$$

(e) The remaining peak interval values in the set $S_4$ are converted to the pulse rate, thereby creating a histogram with intervals of 5 [bpm] of the pulse rate. Under this circumstance, the width of the pulse rate in one section of the histogram is equal, but the width of the peak interval corresponding to that pulse rate varies. So, a histogram with weighted values is created according to Expression (5) indicated below.
[Math. 5]

$$h'_{i-i+5} = \frac{t_{i-i+5}}{t_{30-35}} h_{i-i+5} \qquad \cdots\cdots (5)$$

In the above expression, $t_{30-35}$ represents a time width of the peak interval of the pulse rate from 30 [bpm] to 35 [bpm], $t_{i-i+5}$ represents a time width of the peak interval of the pulse rate from i [bpm] to i+5 [bpm], $h_{i-i+5}$ represents a frequency of the peak interval of the pulse rate from i [bpm] to i+5 [bpm] before the correction, and $h'_{i-i+5}$ represents a corrected value of the frequency of the peak interval of the pulse rate from i [bpm] to i+5 [bpm].

(f) Regarding the value of the weighted histogram, a window of the width 20 [bpm] is provided to maximize the total frequency of adjacent four sections and the peak interval $t_i$ outside that rage is removed.

An average value of the peak interval values extracted according to the above-described algorithms is defined as the peak width t within the measurement time and a value obtained by dividing 60 seconds by the peak interval t is defined as the pulse rate.

Accordingly, the control unit 10 sets the feature points respectively to the top and the bottom of the intensity waveform of the reflected light obtained from the near-infrared detection unit 40 and estimates the pulse rate on the basis of the peak intervals between the tops and between the bottoms of the cycles of the respective feature points. Moreover, the control unit 10 calculates a standard deviation of the plurality of peak intervals and removes the peak intervals which do not indicate the pulse cycles with reference to the standard deviation. Furthermore, the control unit 10 creates the weighted histogram on a specified time basis from the pulse rate converted from the peak intervals which have remained after the removal, and corrects the frequency of the peak interval on the basis of the histogram.

Consequently, the control unit 10 can calculate the accurate pulse rate from the measured waveform of the pulse waveform data of relatively short time based on a pixel group within the set ROI.

(2-1-2) Skin Temperature Measurement by Far-Infrared Detection Unit

Heat generated inside the living body is carried to the body surface by means of conduction and convection. However, the heat conduction by biological tissues themselves is poor and acts as heat insulation, so that most of the heat carriage to the skin is conducted by a skin blood flow. The skin blood flow volume changes due to activities of the autonomic nervous system mainly caused by shrinkage and expansion actions of blood vessels by a sympathetic nervous system and a parasympathetic nervous system.

In prior conventional studies, an attempt has been made to estimate the activities of the autonomic nervous system by measuring changes in the skin temperature. However, since heat distribution of a face is greatly influenced by the measurement subject's hair style and whether the measurement subject wears glasses or not, the active state sensing unit 3 according to the present invention is designed to use the near-infrared image to set an appropriate measurement site for estimating the activities of the autonomic nervous system.

In order to estimate a stressed state from changes in the skin temperature, it is necessary to set a site in the face where temperature changes caused by the stress appear prominently, as the ROI. Arteriovenous anastomoses (AVA) which are susceptible to the activities of the autonomic nervous system are concentrated particularly at a nose part among sites in the face, so that the nose part is suited for the measurement of the skin temperature for the purpose of estimating the stressed state.

Moreover, since the skin temperature is susceptible to the outside temperature, it is necessary to record changes in the skin temperature as a relative temperature. When doing so, the ROI to be measured as a reference for changes in the skin temperature at the nose part needs to be set at a site that is hardly susceptible to the activities of the autonomic nervous system. An example of the site, which has a low concentration degree of AVA and is hardly susceptible to the activities of the autonomic nervous system, in the face is a forehead part.

Accordingly, the control unit 10 according to the present invention records the changes in the skin temperature as a temperature difference between a temperature of the region of interest ROI_n at the nose part and a temperature of the region of interest ROI_fh at the forehead part. Fig. 4(B) illustrates the locations of the respective ROI. Moreover, Expression (6) indicates an expression for calculating the temperature change.

[Math. 6]

$$T_r = T_n - T_{fh} \qquad \cdots\cdots (6)$$

In the above expression, $T_n$ represents an average value of the skin temperature measured at all pixels within the ROI_n, $T_{fh}$ represents an average value of the skin temperature measured at all pixels within the ROI_fh, and $T_r$ represents a relative temperature at the forehead part and the nose part. When setting the respective ROI, coordinates of the nose part and the forehead part in the near-infrared image are transformed to coordinates in a far-infrared image.

Specifically, regarding the coordinate transformation from the near infrared (NIR) image to the far infrared (FIR) image, a near infrared camera and a far infrared camera have different resolutions and angles of view, so that coordinate information of the near-infrared image is associated with pixels of the far-infrared image.

In other words, the coordinate transformation can be expressed as the following Expression (7) indicated below, where $X_{fir}$ represents an x-coordinate in the far-infrared image, $\theta_{nir\_h}$ represents a horizontal angle of view of the near infrared camera, and $\theta_{fir\_h}$ represents a horizontal angle of view of the far infrared camera.

[Math. 7]

$$x_{fir} = \frac{40}{\tan\dfrac{\theta_{fir\_h}}{2}} \left( \frac{\tan\dfrac{\theta_{nir\_h}}{2}}{320} x_{nir} + \tan\frac{\theta_{fir\_h}}{2} - \tan\frac{\theta_{nir\_h}}{2} \right) \quad \cdots\cdots (7)$$

Moreover, the coordinate transformation can be expressed as the following Expression (8) indicated below, where Yfir represents a y-coordinate in the far-infrared image, $\theta_{nir\_v}$ represents a vertical angle of view of the near infrared camera, and $\theta_{fir\_v}$ represents a vertical angle of view of the far infrared camera. Incidentally, d represents the difference in optical axes and L represents the distance to an object.

[Math. 8]

$$y_{fir} = \frac{30}{\tan\dfrac{\theta_{fir\_v}}{2}} \left( \frac{\tan\dfrac{\theta_{nir\_v}}{2}}{240} y_{nir} + \tan\frac{\theta_{fir\_v}}{2} - \tan\frac{\theta_{nir\_v}}{2} - \frac{d}{L} \right) \cdots\cdots (8)$$

Consequently, the control unit 10 can accurately calculate the relative temperature difference in the skin temperature between the forehead part and the nose part of the subject on the basis of the automatic sensing result of the measurement target sites using the image information of the near-infrared detection unit 40, and subsequently also on the basis of the image information of the far-infrared detection unit 41.

(2-1-3) Breathing Measurement Based on Skin Temperature

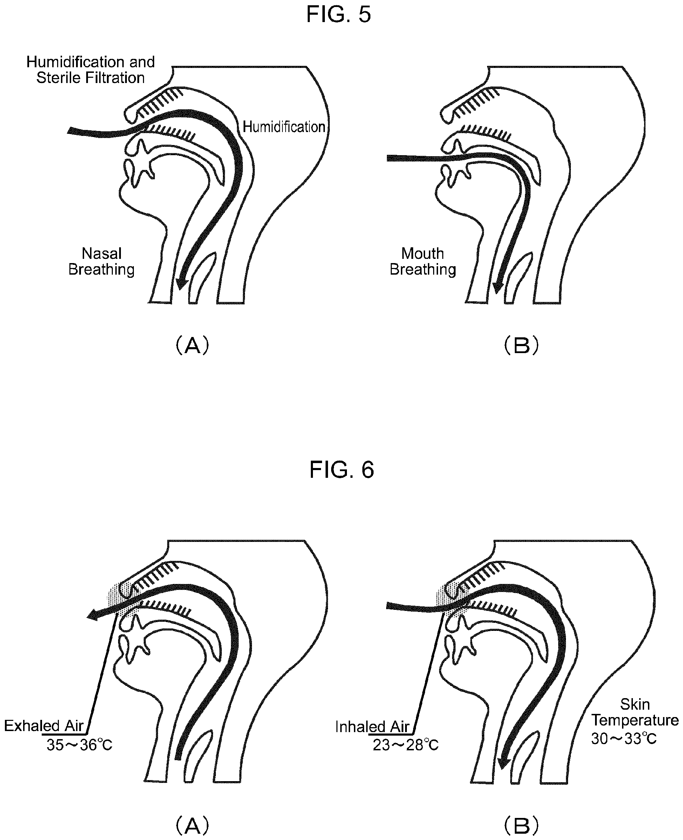

Breathing is related to working and immunity of the autonomic nervous system and is used to diagnose a sleeping disorder. The breathing includes nasal breathing (Fig. 5(A)) and mouth breathing (Fig. 5(B)) and the nasal breathing is desired essentially as breathing. In a case of the mouth breathing, inhalation does not pass through cilia or mucous membranes in a nasal cavity. Moreover, the mouth breathing causes various diseases such as a sleep apnea syndrome (SAS).

Referring to Fig. 6(A) and Fig. 6(B), nostrils and an oral cavity are warmed up by exhalation (35 to 36[°C]) and cooled down by the inhalation (23 to 28[°C]). Therefore, a region of interest (ROI) is set to the subject's nostrils and oral cavity and temperature changes in the nostrils and the oral cavity as caused by breathing are detected by the far-infrared detection unit (far infrared camera) 41. Since a surface temperature of the skin is influenced by the outside temperature, it is measured as a relative temperature between the nostrils and the oral cavity.

Practically, automatic detection of measurement target sites by means of a skin temperature distribution map is difficult, so that the automatic detection of the measurement target sites (the nostrils and the oral cavity) is performed by using the image information of the near-infrared detection unit (camera) as mentioned earlier and the region of interest (ROI) is set. Then, while a nostril temperature during the nasal breathing is relatively high, an oral temperature during the mouth breathing is relatively low.

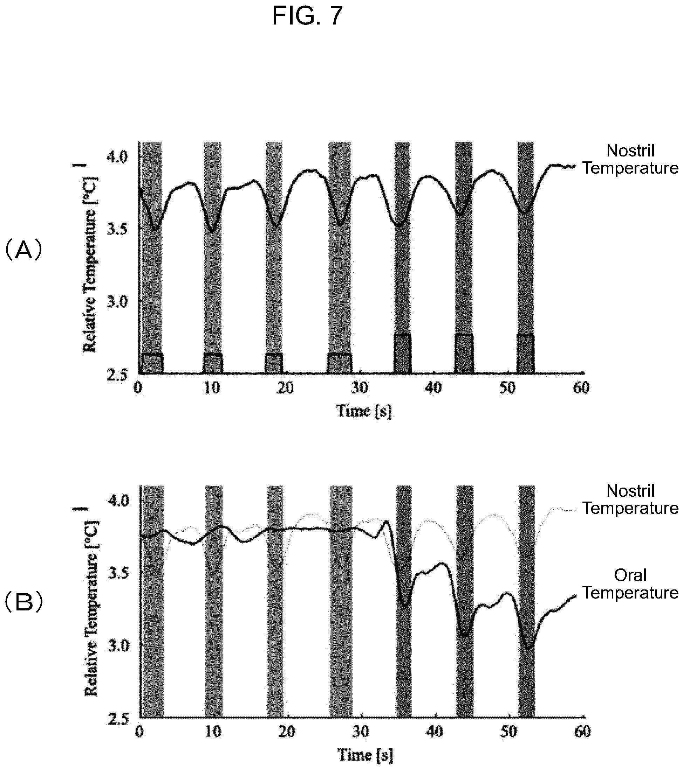

The nasal breathing and the mouth breathing by the subject were actually continuously measured for 30 seconds each, then there was a one-minute break, and the breathing measurement was repeated four times in the same manner. As a result, it was confirmed as illustrated in Fig. 7(A) that the temperature of the nostrils decreased by the inhalation by the nasal breathing. Moreover, it was also confirmed as illustrated in Fig. 7(B) that the temperature of the oral cavity decreased in association with the mouth breathing.

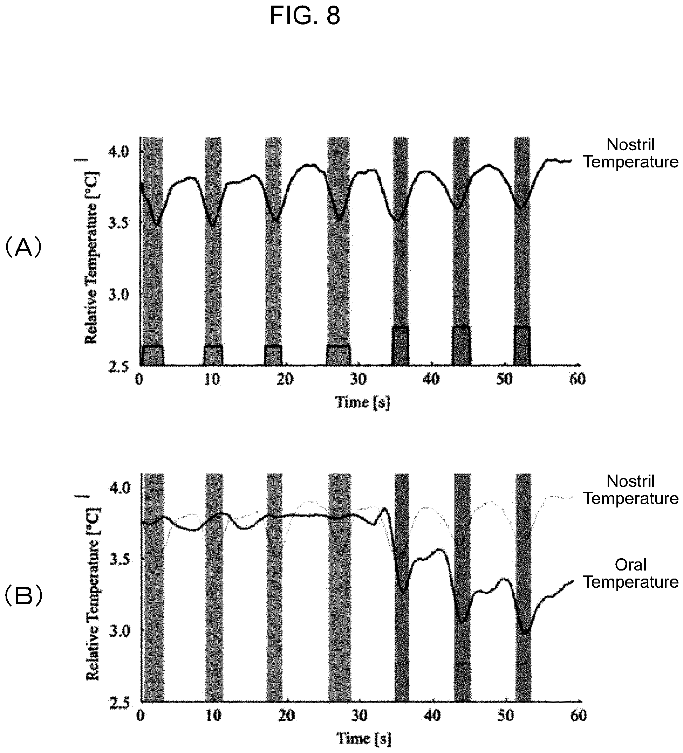

Furthermore, as illustrated in Fig. 8(A), opening and closing of a mouth as caused by a change in the breathing method from the nasal breathing to the mouth breathing were also confirmed based on the distance between an upper lip and a lower lip. The graphs of Fig. 7(A), Fig. 7(B), and Fig. 8(A) are superimposed and displayed in Fig. 8(B).

Incidentally, an inhale timing extraction method is to firstly remove a high-frequency noise from the measurement result of the relative temperature between the nostrils and the oral cavity by using the moving average filter and extract the timing of the temperature decrease by the exhalation by executing first derivation.

Moreover, if the nostril temperature and the oral temperature decrease at the same timing, an intra-alveolar pressure becomes negative pressure relative to the atmospheric pressure and the air flows into a nasal cavity path; and, therefore, it can be determined as the mouth breathing.

Consequently, it is possible to implement the calculation of the breathing rate by the non-contact measurement method and to discriminate the breathing method. The nasal breathing and the mouth breathing can be measured at the same time and it is possible to implement the accurate calculation of the breathing rate and discriminate the breathing method by executing the algorithms in consideration of characteristics of temperature changes upon each breathing.

(2-1-4) Oxygen Saturation Using Two Types of Near-Infrared Wavelengths

An example of conventional measurement equipment which utilizes light absorption property by the hemoglobin in the blood is a pulse oximeter. The pulse oximeter is medical equipment for measuring blood oxygen saturation and the pulse rate by wearing the equipment on a fingertip.

Since the blood oxygen saturation has characteristics which change depending on the breathing rate, the degree of coupling between the hemoglobin and the oxygen, and cardiac outputs, the present invention focuses on the difference in the light absorption characteristics between oxidized hemoglobin ($HbO_2$) and reduced hemoglobin (Hb).

The conventional measurement method uses a method of irradiating the fingertip with infrared light (in the vicinity of wavelength 660 [nm]) and near infrared light (in the vicinity of wavelength 900 [nm]) from a light-emitting element, estimating the blood oxygen saturation from a light quantity ratio of each transmitted light measured by a light-receiving element and, at the same time, estimating a pulse rate by utilizing periodic changes in the light quantity of the transmitted light. The infrared light which is visible light has been utilized because it has a large light absorptivity difference between the oxidized hemoglobin ($HbO_2$) and the reduced hemoglobin (Hb).

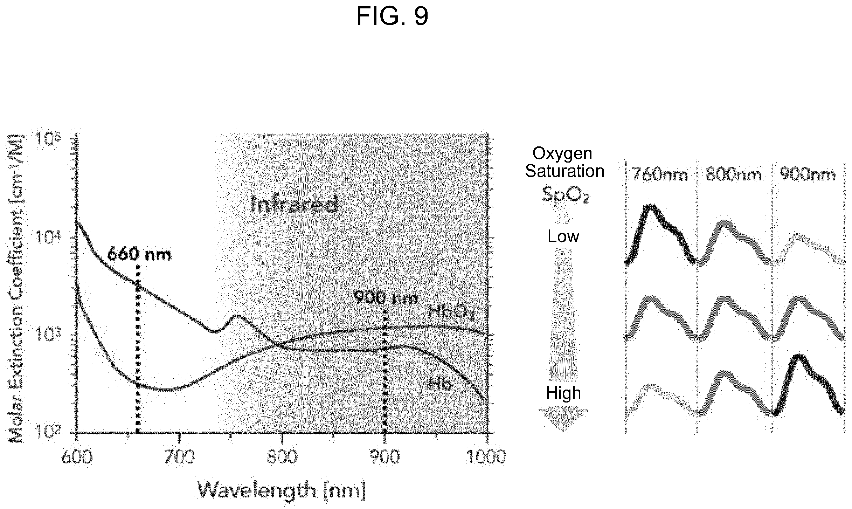

However, the present invention is designed to perform non-contact measurement by using only the near infrared rays (NIR) which is invisible light (Fig. 9). As a result, the blood oxygen saturation of mainly the ROI of the subject can be measured in a non-contact manner and even under a dark environment by causing light-emitting elements which emit two types of the near infrared light with wavelength of 800 (or 760) [nm] and wavelength of 900 [nm] to blink alternately and capturing images of an irradiation site (ROI) of each of these light-emitting elements with one near infrared camera (the near-infrared detection unit 40).

(2-2) Method for Sensing Active State of Nervous System

The autonomic nerves include sympathetic nerves which function when the living body is in a state of tension or the active state, and parasympathetic nerves which function when the living body is in a state of resting quietly in bed. In a state where the sympathetic nerves are predominant, a blood pressure value and a pulse rate rise. On the other hand, in a state where the parasympathetic nerves are predominant, the blood pressure value and the pulse rate fall. So, the autonomic nervous function and the cardiac function have a high correlation.

As a method for measuring the autonomic nervous function, there are methods for measuring the function of the sympathetic nerves of the heart by using heart rate variability, including a CVR-R (Coefficient of Variation of R-R intervals) method of evaluation by finding a variation coefficient by using R-R intervals of the electrocardiogram waveform and secondly a method of using a variable power rate of frequency components (a high frequency component and a low frequency component) of the heart rate variability as an index for the sympathetic nerve activities.

Moreover, there is also a method for measuring the autonomic nervous function by measuring the sympathetic nerve function of a blood vessel system by using pulse waves. An example of this measurement method is particularly a method of calculating the size of amplitude fluctuations of a photoplethysmography (PPG) waveform as an evaluation value of the autonomic nervous function.

According to the present invention, the mobile terminal 2 can contribute to the evaluation of the subject's autonomic nervous function (for example, whether breathing is disordered or not) on the basis of a combination of all or some of the subject's pulse rate, the relative temperature difference in the skin temperature between the subject's forehead part and their nose part, the subject's breathing rate and breathing method, and the subject's oxygen saturation which are detected by using the near-infrared detection unit 40 and the far-infrared detection unit 41 which constitute the active state sensing unit 3.

(2-3) Method for Sensing Active State of Motor System

The active state sensing unit 3 includes the movement recognition unit 42 which recognizes the subject's movements for each action phase when the subject executes a behavior task (Fig. 2). The movement recognition unit 42 includes, as part of its constituent element, an IMU (Inertial Measurement Unit) sensor capable of implementing inertia-type motion capture which is a means of measuring human actions.

The IMU sensor is configured by equipping one chip with an imaging camera, optical motion capture, mechanical motion capture, an acceleration, angular velocity, and geomagnetism sensor and is capable of measuring triaxial acceleration, angular velocity, and geomagnetism.

The inertia-type motion capture is a measurement method which can be used indoors and outdoors and there are no limitations to a measurement location or a shooting range, and a phenomenon which makes it no longer possible to perform normal measurement as a result of disappearance of a necessary marker for measurement of positional information (occlusion) does not occur. So, it is believed to be suited for a method for measuring strenuous actions such as sports.

According to the present invention, a sensor module capable of measuring the acceleration and the angular velocity during high-speed actions by means of high-speed sampling is applied as the movement recognition unit 42. Specifically, the movement recognition unit 42 is composed of a sensor module in which the IMU sensor, a triaxial acceleration sensor, and a monoaxial angular velocity sensor are arranged perpendicularly to each other. Then, this sensor module is designed to switch between the respective sensors according to a measurement range so that their output values remain a linear relationship.

Accordingly, the active state sensing unit 3 senses the motion data recognized by the movement recognition unit 42 as the active state of the subject's motor system.

(2-4) Method for Sensing Active State of Cognitive System

The active state sensing unit 3 includes the recognition degree sensing unit 43 (Fig. 2) which measures a reaction rate of the subject to start an action after recognition by the subject by using at least one or more senses from among their visual sense, auditory sense, and tactile sense for a specified amount of time.

The recognition degree sensing unit 43 adopts a flicker test technique as a method of measuring the above-described reaction rate of the subject. The flicker test technique utilizes a phenomenon where in a state of a light source blinking at a high speed, it is difficult to recognize flickers of the light; however, if a frequency which defines the speed of light flickering is decreased, it becomes possible to recognize the flickers from a certain frequency.

The frequency at which it starts becoming possible to recognize the flickers is considered as a threshold value for recognizing the flickers; and it is known that the threshold value changes along with the mental fatigue. Specifically, the flicker recognition threshold value has characteristics such that the threshold value decreases along with the fatigue, it becomes impossible to recognize the light flickering at high frequency, and the light flickers at a frequency lower than that of the normal healthy condition can only be recognized.

Consequently, the active state detection unit 3 detects the subject's recognition degree as the active state of the subject's cognitive system in accordance with the measurement result (recognition result) by the recognition degree sensing unit 43.

(3) Other Embodiments

Incidentally, the aforementioned embodiment has described the case where the following are applied as the active state sensing unit 3: the near-infrared detection unit 40 and the far-infrared detection unit 41 which mainly sense the active state of the physiological system and the nervous system, such as a blood pressure meter, a heartbeat sensor, a weight scale, a thermometer, a sleep apnea treatment device (CPAP), a blood sugar level measurement device, and a pace maker which are medical equipment; the movement recognition unit 42 which mainly senses the active state of the motor system; and the recognition degree sensing unit 43 which mainly senses the active state of the cognitive system. However, the present invention is not limited to this example and various sensors may be applied as long as they are capable of communicating with the mobile terminal via the short-range wireless communication.

For example, the heart beat sensor is a sensor which operates in contact with a human body such as an arm (where there are blood vessels) by sensing a blood flow and is designed to sense reflected light, which is obtained by emitting light of two green LEDs (wavelength: 570 [nm]), with a photodiode.

Moreover, this embodiment has described the case where a smartphone is applied as the mobile terminal 2; however, the present invention is not limited to this example and can be applied to various mobile terminals, such as a tablet and a hand-held terminal, as long as they are capable of communicating with the active state sensing unit via the short-range wireless communication.

Furthermore, this embodiment has described the case where the Bluetooth communication standards are applied as the short-range wireless communication by the communication interface units 11, 20; however, the present invention is not limited to this example and may apply various communication standards such as wireless LAN communication having a pairing connection function, mobile communication networks such as 3G/LTE, and NFC (Near Field Communication).

REFERENCE SIGNS LIST

| | |
|---|---|
| 1: | biological information management system |
| 2: | mobile terminal |
| 3: | active state sensing unit |
| 4: | communication network |
| 5: | server apparatus |
| 10: | control unit |
| 11, 20: | communication interface units |
| 13: | display unit |
| 30: | control apparatus |
| 31: | database |
| 40: | near-infrared detection unit |
| 41: | far-infrared detection unit |
| 42: | movement recognition unit |
| 43: | recognition degree sensing unit |

**Claims**

1. A biological information management system comprising:

an active state sensing unit that senses an active state of at least one or more systems of a subject from among active states of the subject's cognitive system, motor system, nervous system, and physiological system;
a mobile terminal capable of communicating with the active state sensing unit via short-range wireless communication;
a communication interface unit provided at each of the active state sensing unit and the mobile terminal and designed to establish pairing between the active state sensing unit and the mobile terminal by means of personal authentication; and
a server apparatus capable of communicating with the mobile terminal via a communication network,
wherein the mobile terminal includes:
a control unit that generates a command signal for causing the active state sensing unit to execute a desired function in response to the subject's operation and transmits the command signal to the active state sensing unit with which the pairing is established via the communication interface unit; and
a data storage unit that stores active state data which is a sensing result of the active state sensing unit which is received via the communication interface unit; and
wherein the control unit reads the active state data, which is stored in the data storage unit, for each specified period and transmits the active state data to the server apparatus via the communication network.

2. The biological information management system according to claim 1,
wherein with the mobile terminal, the control unit stores an application according to the active state sensing unit, which is a pairing target, reads the application data which corresponds to the active state sensing unit from the active state sensing unit at the time of establishment of the pairing with the active state sensing unit, and activates the application based on the application data.

3. The biological information management system according to claim 1 or 2,
wherein the mobile terminal further includes a display unit that displays a function screen corresponding to the application when with the mobile device the control unit activates the application according to the active state sensing unit with which the pairing has been established.

4. The biological information management system according to claim 3,
wherein with the mobile terminal, the control unit displays the active state based on the active state data, which is the sensing result of the active state sensing unit received via the communication interface unit, on a real-time basis by combining the active state with the function screen of the display unit.

5. The biological information management system according to any one of claims 1 to 4, wherein with the mobile terminal, the control unit: causes the display unit to display an application for inputting measurement content regarding a body of the subject; and causes the data storage unit to store a measurement result regarding the body of the subject as physical data together with personal information of the subject.

6. A biological information management method when an active state sensing unit that senses an active state of at least one or more systems of a subject from among active states of the subject's cognitive system, motor system, nervous system, and physiological system is made capable of communicating with a mobile terminal via short-range wireless communication and the mobile terminal is made capable of communicating with a server apparatus via a communication network, the biological information management method comprising:

   a first step of establishing pairing between the active state sensing unit and the mobile terminal by means of personal authentication via a communication interface unit provided at each of the active state sensing unit and the mobile terminal;
   a second step executed at the mobile terminal generating a command signal for causing the active state sensing unit to execute a desired function in response to the subject's operation and transmitting the command signal to the active state sensing unit with which the pairing is established via the communication interface unit;
   a third step executed at the mobile terminal storing active state data, which is a sensing result of the active state sensing unit which is received via the communication interface unit, in a data storage unit; and
   a fourth step executed at the mobile terminal reading the active state data, which is stored in the data storage unit, for each specified period and transmitting the active state data to the server apparatus via the communication network.

7. The biological information management method according to claim 5,
   wherein in the second step at the mobile terminal, an application according to the active state sensing unit, which is a pairing target are stored in the data storage unit, the application data which corresponds to the active state sensing unit is read from the active state sensing unit at the time of establishment of the pairing with the active state sensing unit, and the application based on the application data is activated.

8. The biological information management method according to claim 5 or 6,
   wherein in the second step at the mobile terminal, a function screen corresponding to the application is displayed on a display unit when with the mobile terminal the application according to the active state sensing unit with which the pairing has been established is activated.

9. The biological information management method according to claim 7,
   wherein in the second step at the mobile terminal, the active state based on the active state data, which is the sensing result of the active state sensing unit received via the communication interface unit, is displayed on a real-time basis by combining the active state with the function screen of the display unit.

10. The biological information management method according to any one of claims 6 to 9,

    wherein in the second step at the mobile terminal, an application for inputting measurement content regarding a body of the subject is displayed on the display unit; and
    a measurement result regarding the body of the subject is stored, as physical data together with personal information of the subject, in the data storage unit.

# FIG. 1

1 Biological Information Management System

**Mobile Terminal** — 2

Active State Sensing Unit (Sensor Group) — 3
Communication I/F — 20

Control Unit — 10
Display Unit — 13
Communication I/F — 11
Data Storage Unit — 12

4

**Server Apparatus** — 5
Control Apparatus — 30
Database — 31

FIG. 2

```
                                              ⌇ 3
┌──────────────────────────────────────┐
│         Active State Sensing Unit      │
│                                        │
│   ┌──────────────────────┐             │
│   │   Near-Infrared      │  ⌇ 40       │
│   │   Detection Unit     │             │
│   └──────────────────────┘             │
│                                        │
│   ┌──────────────────────┐             │
│   │   Far-Infrared       │  ⌇ 41       │
│   │   Detection Unit     │             │
│   └──────────────────────┘             │
│                                        │
│   ┌──────────────────────┐             │
│   │   Movement           │  ⌇ 42       │
│   │   Recognition Unit   │             │
│   └──────────────────────┘             │
│                                        │
│   ┌──────────────────────┐             │
│   │   Recognition        │  ⌇ 43       │
│   │   Degree Sensing     │             │
│   │   Unit               │             │
│   └──────────────────────┘             │
└──────────────────────────────────────┘
```

# FIG. 3

(A)

(B)

# FIG. 4

(A)

ROI

(B)

ROI_fh

ROI_n

FIG. 5

Humidification and
Sterile Filtration

Humidification

Nasal
Breathing

(A)

Mouth
Breathing

(B)

FIG. 6

Exhaled Air
35~36°C

(A)

Inhaled Air
23~28°C

Skin
Temperature
30~33°C

(B)

# FIG. 7

(A)

(B)

FIG. 8

（A）

Nostril
Temperature

（B）

Nostril
Temperature

Oral
Temperature

FIG. 9

## INTERNATIONAL SEARCH REPORT

| International application No. |
|---|
| PCT/JP2021/027857 |

### A. CLASSIFICATION OF SUBJECT MATTER
Int.Cl. G16H10/00(2018.01)i
FI: G16H10/00

According to International Patent Classification (IPC) or to both national classification and IPC

### B. FIELDS SEARCHED

Minimum documentation searched (classification system followed by classification symbols)
Int.Cl. G16H10/00

Documentation searched other than minimum documentation to the extent that such documents are included in the fields searched
Published examined utility model applications of Japan     1922-1996
Published unexamined utility model applications of Japan     1971-2021
Registered utility model specifications of Japan     1996-2021
Published registered utility model applications of Japan     1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2004-147705 A (MATSUSHITA ELECTRIC WORKS, LTD.) 27 May 2004 (2004-05-27), paragraphs [0023]-[0056] | 1-10 |
| Y | JP 2020-119433 A (FUJI XEROX CO., LTD.) 06 August 2020 (2020-08-06), paragraphs [0029], [0043] | 1-10 |
| Y | WO 2014/185487 A1 (SHARP CORPORATION) 20 November 2014 (2014-11-20), paragraph [0069] | 2, 4, 5, 7, 9, 10 |
| Y | JP 2017-174168 A (FINC INC.) 28 September 2017 (2017-09-28), paragraphs [0015], [0033], fig. 5 | 4, 5, 9, 10 |

☐ Further documents are listed in the continuation of Box C.          ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 30 August 2021 | 07 September 2021 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

| INTERNATIONAL SEARCH REPORT<br>Information on patent family members | International application No.<br>PCT/JP2021/027857 |
|---|---|

| | | |
|---|---|---|
| JP 2004-147705 A | 27 May 2004 | (Family: none) |
| JP 2020-119433 A | 06 August 2020 | US 2020/0245015 A1<br>paragraphs [0038], [0090]-[0094]<br>CN 111488781 A |
| WO 2014/185487 A1 | 20 November 2014 | JP 2014-229942 A<br>paragraph [0042]<br>JP 2014-228898 A<br>JP 2014-228899 A |
| JP 2017-174168 A | 28 September 2017 | (Family: none) |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017012604 A, Kokai

- JP 2018059938 A, Kokai